# EUROPEAN PATENT APPLICATION

(11) **EP 3 971 160 A1**
(43) Date of publication of application: **23.03.2022**
(21) Application number: 20806271.1
(22) Date of filing: 13.05.2020
(51) Int. Cl.: C07C 51/41, C07C 57/15, C07C 57/22, C07C 63/24, C07C 63/26, C07C 63/307, C07C 65/03, C07D 233/58, C07F 1/08, C07F 3/02, C07F 3/04, C07F 3/06, C07F 5/06, C07F 11/00, C07F 15/02, C07F 7/00, C07D 213/79

(54) **METHOD FOR PRODUCING METAL-ORGANIC STRUCTURE**

(30) Priority: 14.05.2019 JP 2019091542
(71) Applicant: Atomis Inc., Kyoto-shi, Kyoto 602-0841 (JP)
(72) Inventor: ASARI, Daisuke, Kyoto-shi Kyoto 602-0841 (JP); KATAOKA, Dai, Kyoto-shi Kyoto 602-0841 (JP)
(74) Representative: AOMB Polska Sp. z.o.o.
(86) International application number: PCT/JP2020/019130
(87) International publication number: WO 2020/230820

(57) **Abstract**

An object of the present invention is to produce a high-quality Metal-Organic Framework in a short time. A method for producing a Metal-Organic Framework according to the present invention includes simultaneously and continuously applying centrifugal force and shear force to a formulation containing a metal ion donor, a multidentate ligand, and a solvent.

## Description

### Field of the Invention:

The present disclosure relates to a method for producing Metal-Organic Frameworks (MOFs).

### Background:

A group of substances called Metal-Organic Framework has been attracting attention in fields such as gas storage and gas separation. The Metal-Organic Framework is a compound having a structure in which metal atoms are crosslinked with each other by an organic ligand, and typically has porosity. Metal-Organic Frameworks with porosity are also called Porous Coordination Polymers (PCPs).

A liquid phase synthesis method such as a solution method, a hydrothermal method, a microwave method, and an ultrasonic method has been typically used as a method for producing the Metal-Organic Framework. A solid phase synthesis method using a mortar, a ball mill or the like has also been used. In recent years, a method of synthesizing Metal-Organic Frameworks using a biaxial mixing apparatus called an extruder has also been reported. In this method, a Metal-Organic Framework is produced by admixing a first reactant containing a specific metal ion donor and a second reactant containing a specific organic ligand under conditions of prolonged and sustained pressure and shear sufficient to synthesize the Metal-Organic Framework (Patent Literature 1)

### Citation List

### Patent Literature

[Patent Literature 1] US 9,815,222 B2

### Summary of the Invention:

### Technical Problem

However, the present inventors have found that it is sometimes difficult to synthesize a high-quality Metal-Organic Framework in a short reaction time when the above methods are used. It is therefore an object of the present invention to provide a method for producing a high-quality Metal-Organic Framework in a short time.

### Solution to Problem

The present inventors have conducted diligent studies in order to solve the above problems. As a result, the present inventors have found a new means of applying to material synthesis a technique conventionally used exclusively for dispersing and/or atomizing particles or droplets.

Some aspects of the present invention are as described below.
[1] A method for producing a Metal-Organic Framework, comprising: simultaneously and continuously applying a centrifugal force and a shear force to a formulation containing a metal ion donor, a multidentate ligand, and a solvent.
[2] The method according to [1], wherein the solvent is a poor solvent for at least one of the metal ion donor and the multidentate ligand.
[3] The method according to [1] or [2], wherein an amount of the solvent is in a range of 30 to 2000% by weight based on a total amount of the metal ion donor and the multidentate ligand.
[4] The method according to any one of [1] to [3], wherein the method is carried out at a temperature lower than a normal boiling point of the solvent.
[5] The method according to any one of [1] to [4], wherein the method is carried out while supplying at least one gas selected from the group consisting of dry air, argon, nitrogen, and oxygen into a reaction vessel.
[6] The method according to any one of [1] to [5], wherein the centrifugal force is generated by stirring the formulation by rotating a rotary blade within a reaction vessel, and the shear force is generated by contact between the formulation and an inner wall of the reaction vessel due to the stirring, or by contact between particles constituting the formulation due to the stirring.
[7] The method according to any one of [1] to [6], wherein the centrifugal force and the shear force are applied to the formulation by a thin film swirl mixing method.
[8] The method according to any one of [1] to [7], wherein the Metal-Organic Framework is a Porous Coordination Polymer.

### Advantageous Effects of Invention

The present invention makes it possible to produce a high-quality Metal-Organic Framework in a short time.

### Brief Description of the Drawings:

FIG. 1 is a cross-sectional view schematically illustrating an example of a reactor used in a production method according to an aspect of the present invention.
FIG. 2 is a cross-sectional view schematically illustrating an example of a reactor used in a production method according to another aspect of the present invention.

### Detailed Description of the Invention:

The production methods according to an embodiment of the present invention will hereinafter be described. When referring to the drawings, the same reference numerals are given to the components exhibiting the same or similar functions, and duplicate description will be omitted.

A method for producing a Metal-Organic Framework according to an embodiment of the present disclosure includes simultaneously and continuously applying a centrifugal force and a shear force to a formulation containing a metal ion donor, a multidentate ligand, and a solvent. The production method may comprise the steps of: preparing a formulation containing a metal ion donor, a multidentate ligand, and a solvent; and mixing the formulation while simultaneously and continuously applying a centrifugal force and a shear force to the formulation. Alternatively, this production may be carried out by sequentially adding the materials comprising the above-mentioned formulation into a reactor.

There are no particular restrictions on the type of Metal-Organic Frameworks (MOFs) to be produced. Appropriately combining the type and coordination number of the metal ion with the type and topology of the multidentate ligand leads to a MOF with a desired structure. The MOF may contain two or more types of metal elements, and may contain two or more types of multidentate ligands. The MOF may further contain monodentate ligand(s). The MOF may be porous. In other words, the MOF may be a Porous Coordination Polymer (PCP).

Specific examples of the MOF include those listed in the literatures below:
Reference 1: Yabing He et al., Methane Storage in Metal-Organic Frameworks, Chem Soc Rev, 2014
Reference 2: Jarad A. Mason et al., Evaluating metal-organic frameworks for natural gas storage, Chem. Sci., 2014, 5, 32-51
Reference 3: WO2019/026872

As described above, the formulation used as a raw material for the MOF contains a metal ion donor, a multidentate ligand, and a solvent. As the metal ion donor and the multidentate ligand, any substance can be used as long as it is suitable as a combination for synthesizing a MOF.

The metal elements of the metal ion donor can be, for example, any elements belonging to alkali metals (Group 1), alkaline earth metals (Group 2), or transition metals (Groups 3 to 12). The metal element is typically selected from the group consisting of magnesium, calcium, iron, aluminum, zinc, copper, nickel, cobalt, zirconium, and chromium. The metal ion donor may contain a plurality of metal elements. Alternatively, a plurality of metal ion donors containing different metal elements may be used in combination.

As the metal ion donor, a metal salt is typically used. The metal ion donor may be an organic salt or an inorganic salt. The metal ion donor is typically selected from the group consisting of hydroxides, carbonates, acetates, sulfates, nitrates and chlorides. A plurality of metal ion donors containing the same metal element may be used in combination.

The multidentate ligand is typically an organic multidentate ligand and is preferably selected from the group consisting of carboxylic acid anions, amine compounds, sulfonic acid anions, phosphate anions, and heterocyclic compounds. Examples of the carboxylic acid anion include dicarboxylic acid anion and tricarboxylic acid anion. Specific examples include anions of citric acid, malic acid, terephthalic acid, isophthalic acid, trimesic acid, and derivatives thereof. Examples of the heterocyclic compound include bipyridine, imidazole, adenine, and derivatives thereof.

The type of solvent contained in the above formulation is not particularly limited, and a solvent generally used for synthesizing a MOF can be used. However, the solvent may preferably be a poor solvent for at least one of the metal ion donor and the multidentate ligand. With such a configuration, the formulation does not become a complete solution but becomes a semi-solid, typically a slurry, with solids remaining. This makes it possible to more effectively apply centrifugal force and shear force, which will be described later, to the above-mentioned formulation. Here, the term "poor solvent" for an object means that the solubility in a solvent of the object is 1g/50mL (=20g/L) or less at 25 °C and at atmospheric pressure. Examples of solvents that can be used include water, alcohols such as methanol and ethanol, carboxylic acids such as formic acid and acetic acid, amides such as N, N-dimethylformamide (DMF) and N, N-diethylformamide (DEF), and esters such as ethyl acetate. A mixture of a plurality of solvents may also be used.

The amount of the solvent based on the total amount of the metal ion donor and the multidentate ligand is, for example, in a range of 30 to 2000% by weight, preferably in a range of 100 to 1000% by weight. Adopting such a configuration makes it possible to improve the production efficiency of the MOF.

The above formulation may further contain additional substances such as reaction accelerators. The reaction accelerators are, for example, a basic substance or an acidic substance, and preferably a basic substance. Examples of the basic substance include diethylamine, triethylamine, 2,6-lutidine, pyridine, imidazole, potassium hydroxide, and sodium hydroxide. Examples of acidic substance include formic acid, acetic acid, trifluoroacetic acid, sulfuric acid, nitric acid, hydrochloric acid, and phosphoric acid. As an additional substance, a plurality of reaction accelerators may be used in combination. As an additional substance, a reaction control agent may also be added.

The above formulation is mixed while simultaneously and continuously applying centrifugal force and shear force. This makes it possible to produce a MOF in a short time and with high quality.

Metal-Organic Frameworks tend to be slightly brittle when compared to ordinary organometallic compounds, as implied by the term "framework" therein. Therefore, it is difficult to produce a high-quality MOF without devising a specific production method. For example, in the case of a solid-phase synthesis method using a ball mill device or the like, an extremely strong force is intermittently applied to the raw material. Therefore, the quality of the resulting MOF varies widely. Further, even in the synthesis method using an extruder, as an extremely strong shear force is locally applied to the raw material under high pressure, the similar problem is likely to occur.

Therefore, the present inventors have considered that the above problem may be solved by simultaneously and continuously adding a centrifugal force and a shear force to the above formulation. Conventionally, such a method has been used exclusively for the purpose of dispersing and/or atomizing particles and droplets, and has not been used for material synthesis. However, the present inventors have found that the MOF can be produced in a short time and with high quality by diverting the above method to the production of the MOF.

Examples of the method of simultaneously and continuously applying centrifugal force and shear force to the above-mentioned formulation include the following. Initially, the formulation is introduced into a reaction vessel. Next, a rotary blade provided in the reaction vessel is rotated to stir the formulation at high speed. This rotation imparts centrifugal force to the formulation. Then, by this centrifugal force, the above-mentioned compound is pressed against an inner wall of the reaction vessel. The contact between such a formulation and the inner wall of the reaction vessel imparts a shear force to the formulation. In this way, both centrifugal force and shear force are applied to the formulation simultaneously and continuously. In this state, the metal ion donor in the formulation reacts with the multidentate ligand to obtain a MOF. The shearing force may also be generated when the particles constituting the formulation come into contact with each other.

When the above method is used, the rotation axis of the rotary blade is preferably parallel to the direction of gravity. In this case, the unevenness of the centrifugal force and the shear force applied to the formulation by rotation is reduced as compared with, for example, the case where the rotation axis is perpendicular to the gravity direction.

One example of such a method is the thin film swirl mixing method developed by Primix Corporation. In this method, by using a thin film swirling high-speed mixer, centrifugal force and shear force can be simultaneously and continuously applied to the introduced substance. As a result, in the conventional use example, particles and droplets are dispersed and/or atomized. Specific device configurations are disclosed, for example, in JPA2007-125454.

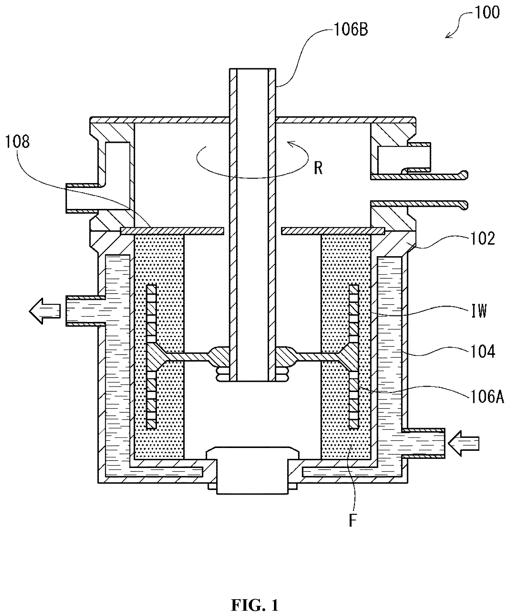

FIG. 1 is a cross-sectional view schematically illustrating an example of a reactor used in a production method according to an aspect of the present invention. The reactor 100 shown in FIG. 1 is a batch type manufacturing device.

The reactor 100 includes a reaction vessel 102. The reaction vessel 102 is, for example, cylindrical. The reaction vessel 102 typically includes an outer layer 104 for temperature control. The outer layer 104 is configured so that a liquid such as water can be injected. This makes it possible to control the temperature in the reaction vessel 102, particularly the temperature of the inner wall IW, which will be described later.

The reactor 100 includes a rotary blade 106A and a rotary shaft 106B connected thereto inside the reaction vessel 102. The rotary blade 106A can be configured to rotate by the rotation R of the rotary shaft 106B. The rotary blade 106A is, for example, a cylindrical wheel having a slight gap with the inner wall IW of the reaction vessel 102. The wheel is typically provided with a number of holes for the formulation F to pass through.

A dam 108 is provided on the upper side of the reactor 100. This prevents the reactants from leaking to the upper part of the reactor 100.

In the production method using the reactor 100, initially, the formulation F is introduced into the reaction vessel 102. Next, the formulation F is stirred by rotating the rotary blade 106A through the rotary shaft 106B. Due to the centrifugal force applied to the formulation F, the formulation F is pressed against the inner wall IW while rotating. As a result, not only the above-mentioned centrifugal force but also a steady shearing force is applied to the formulation F. In this way, the formulation F is mixed while simultaneously and continuously applying centrifugal force and shear force. After completion of the reaction, the reaction product is recovered to obtain a desired MOF.

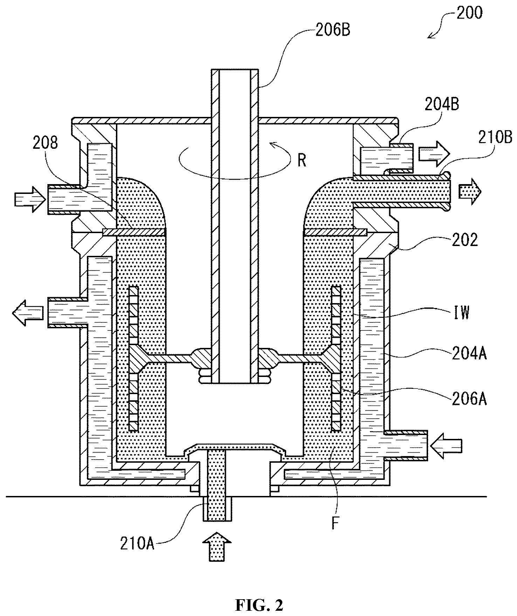

FIG. 2 is a cross-sectional view schematically illustrating an example of a reactor used in a production method according to another aspect of the present invention. The reaction device 200 shown in FIG. 2 is a continuous type manufacturing device.

The reactor 200 includes a reaction vessel 202. The reaction vessel 202 is provided with two outer layers for temperature control. Specifically, in addition to the outer layer 204A having the similar structure as the outer layer 104, an additional outer layer 204B is provided on the upper part of the reaction vessel 202. This makes it possible to control the reaction temperature even in the upper part of the reaction vessel 202.

The reactor 200 includes a rotary blade 206A and a rotary shaft 206B connected thereto inside the reaction vessel 202. The configurations of the rotary blade 206A and the rotary shaft 206B are the same as those described for the rotary blade 106A and the rotary shaft 106B, respectively.

A dam 208 is provided on the upper side of the reactor 200. The dam 208 is smaller in size than the dam 108. This allows at least a portion of the reaction product to be delivered to the upper part of the reactor 200.

The reactor 200 includes an injection port 210A and a discharge port 210B. The injection port 210A is provided in the lower part of the reactor 200 through which the formulation F can be continuously injected. The discharge port 210B is provided in the upper part of the reactor 200, through which at least a part of the reaction product can be discharged to the outside of the system.

In the production method using the reactor 200, initially, the formulation F is introduced into the reaction vessel 202 through the injection port 210A. Next, the formulation F is stirred by rotating the rotary blade 206A through the rotary shaft 206B. Due to the centrifugal force applied to the formulation F, the formulation F is pressed against the inner wall IW while rotating. As a result, not only the above-mentioned centrifugal force but also a steady shearing force is applied to the formulation F. In this way, the formulation F is mixed while simultaneously and continuously applying centrifugal force and shear force. The reaction product obtained by this mixing is discharged from the discharge port 210B as the reaction progresses. By recovering the reaction product discharged in this way, a desired MOF is obtained.

Specific devices for enabling the above-mentioned manufacturing method include, for example, FILMIX (Primix Corporation), Apex Disperser ZERO (Hiroshima Metal & Machinery Chemtech Co., Ltd.), and High-Shear Mixer (SILVERSON). Any device other than these may be used as long as it can simultaneously and continuously apply both centrifugal force and shear force to the formulation.

The above production is preferably carried out while controlling the reaction temperature. In that case, the mixing is preferably carried out at a temperature lower than the normal boiling point of the solvent. The mixing is carried out, for example, at a temperature of 80°C or lower, preferably 60°C or lower. In this way, it is possible to produce MOF in a state in which the solvent in the formulation remains in appropriate amount.

The production can be carried out while supplying at least one gas selected from the group consisting of dry air, argon, nitrogen, and oxygen into the reaction vessel. That is, in the production method according to one embodiment of the present invention, the reaction can be carried out in a closed system. For example, by performing the above production in an atmosphere of an inert gas such as dry air, argon, and nitrogen, it is possible to produce a moisture sensitive MOF with high accuracy. Alternatively, by performing the above production in an oxygen atmosphere, it becomes possible to produce a MOF that is preferably synthesized in an oxygen excess atmosphere with high accuracy.

Further, the above production can be carried out, for example, by mixing the formulation at a linear velocity in the range of 1 to 100m/s, preferably at a linear velocity of 10 to 50m/s. If the linear velocity is too low, it may not be possible to sustainably apply shear forces to the formulation. If the linear velocity is too high, the centrifugal and shear forces applied to the formulation may be excessive.

### EXAMPLES

### Examples 1-38: Centrifugal Shear Synthesis

The metal ion donor, multidentate ligand, solvent, and optionally reaction accelerator shown in Table 1 were added to a thin film swirling high-speed mixer (FILMIX 56-L type; manufactured by Primix Corporation). Next, high-speed stirring was performed under the reaction conditions shown in Table 1. The MOFs were thereby obtained.

### Comparative Examples A1 to A9: Solvothermal synthesis

The metal ion donor, multidentate ligand, solvent, and optionally reaction accelerator shown in Table 2 were added to a 100mL high pressure reaction vessel (HU-100, manufactured by SAN-AI Kagaku Co. Ltd.). Next, solvothermal synthesis was carried out using a constant temperature oven (OFP-300V; manufactured by AS ONE Corporation) under the reaction conditions shown in Table 2.

### Comparative Examples B1 to B10: Ball-Mill Synthesis

The metal ion donor, multidentate ligand, solvent and optionally reaction accelerator shown in Table 3 were added to the 125mL grind jar. A stainless-steel crushing ball having a diameter of 5mm was added thereto, and ball-mill synthesis was carried out using a high-energy ball mill device (Emax; manufactured by Retsch) under the reaction conditions shown in Table 3.

### Comparative Examples C1 to C7: Biaxial kneading synthesis

The metal ion donor and multidentate ligand shown in Table 4 were placed in a polyethylene bag and mixed thoroughly. Then, the mixture was taken out into a stainless-steel container, the solvent shown in Table 4 was added, and the mixture was further stirred and mixed. This was added to a twin-screw kneader (Process 11; manufactured by Thermo Fisher Scientific Co., Ltd.), and biaxial kneading synthesis was carried out under the reaction conditions shown in Table 4.

### Evaluation

The sample obtained by each of the above methods was dried under reduced pressure for 24 hours at room temperature using a vacuum desiccator (MVD-300; manufactured by AS ONE Corporation). The dried sample was subjected to XRD measurement using an X-ray diffractometer (MiniFlex; manufactured by Rigaku Co., Ltd.). Further, at least some of the samples were heated and vacuum-dried at 140°C for 4 hours using a gas adsorption pretreatment apparatus (BERPREP-vacIII; MicrotracBEL), and then the BET specific surface area (N₂; 77K) was measured using a gas adsorption apparatus (BELSORP-miniX; MicrotracBEL).

The quality of the obtained Metal-Organic Framework was evaluated by the presence or absence of a crystalline peak by XRD measurement and the size of the BET specific surface area S_{BET}. These results are summarized in Tables 1 to 4.

The following abbreviations are used in Tables 1 to 4.
BTC: 1,3,5-benzenetricarboxylic acid (trimesic acid)
pBDC: terephthalic acid
iBDC: isophthalic acid
INA: 4-pyridinecarboxylic acid
Mim: 2-methylimidazole
ADC: acetylenedicarboxylic acid
DOT: dihydroxyterephthalic acid
Fumarate: fumaric acid
BTC3Na: trisodium 1,3,5-benzenetricarboxylate
Further, ethanol (EtOH) having a purity of 99.5% or more was used.

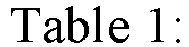

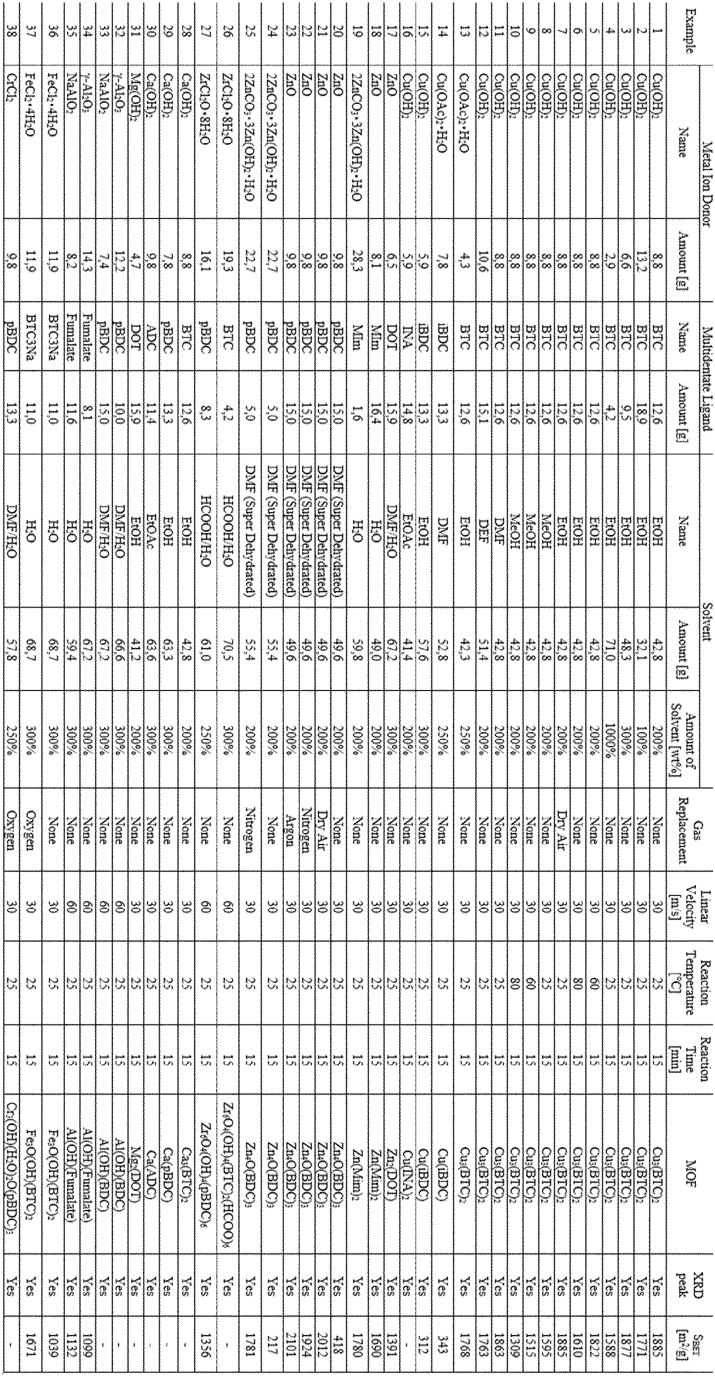

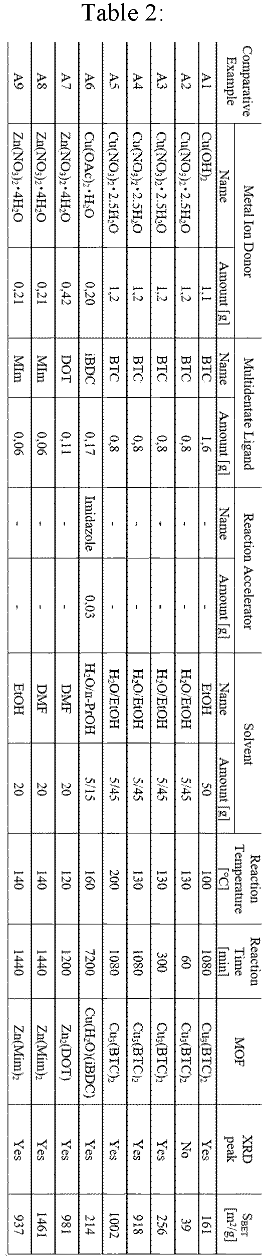

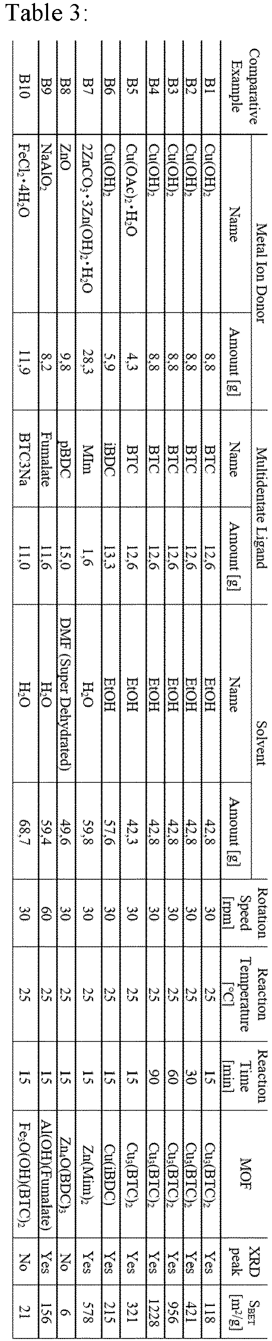

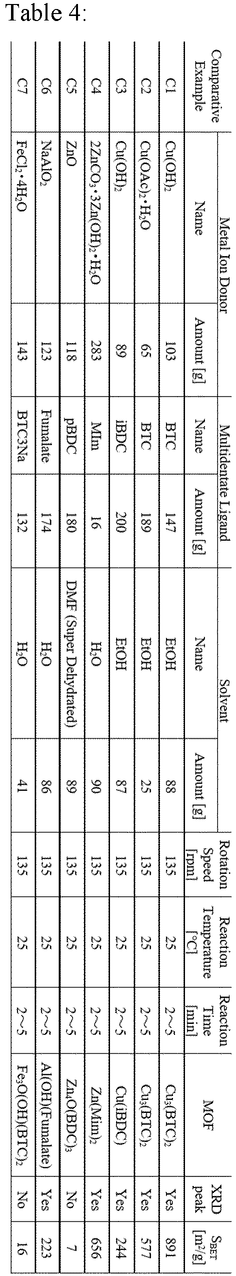

Comparing Tables 1 and 2, it can be seen that by using the production method according to the present invention, higher quality MOFs can be synthesized while significantly shortening the reaction time compared with the case of using the conventional solvothermal method. Comparing Table 1 with Tables 3 and 4, it can be seen that by using the production method according to the present invention, higher quality MOFs can be synthesized with a similar reaction time, which is comparable to the case of using the conventional ball-mill method and the biaxial kneading method.

Further, when Examples 1 to 4 are compared as in Table 1, it can be seen that the specific surface area of the obtained MOF varies by controlling the amount of the solvent. This result suggests that the centrifugal force and shear force applied to the formulation can be optimized by controlling the viscosity of the formulation by adjusting the amount of solvent.

Further, in Table 1, when Examples 1, 5 and 6 or Examples 8 and 9 are compared, it can be seen that higher quality MOF can be synthesized by performing the reaction at a temperature below the normal boiling point of the solvent (78.4°C in the case of ethanol, 64.7°C in the case of methanol), preferably below 60°C.

Further, comparing Examples 20 to 23 in Table 1, it can be seen that a higher quality MOF can in some cases be synthesized by carrying out the reaction in a dry air, nitrogen, or argon atmosphere. Similarly, comparing Examples 24 and 25 in Table 1, it can be seen that a higher quality MOF can in some cases be synthesized by performing the reaction in a nitrogen atmosphere. Furthermore, comparing Examples 36 and 37 in Table 1, it can be seen that a higher quality MOF can in some cases be synthesized by performing the reaction in an oxygen atmosphere. In this way, by carrying out the reaction in a closed system as needed, it becomes possible to synthesize a wider range of MOFs.

### DESCRIPTION OF SYMBOLS

100: Reactor, 102: Reaction Vessel, 104: Outer Layer, 106A: Rotary Blade, 106B: Rotary Shaft, 108: Dam, 200: Reactor, 202: Reaction Vessel, 204A: Outer Layer, 204B: Outer Layer, 206A: Rotary Blade, 206B: Rotary Shaft, 208: Dam, 210A: Injection Port, 210B: Discharge Port, F: Formulation, IW: Inner Wall, R: Rotation.

## Claims

1. A method for producing a Metal-Organic Framework, comprising: simultaneously and continuously applying a centrifugal force and a shear force to a formulation containing a metal ion donor, a multidentate ligand, and a solvent.

2. The method according to claim 1, wherein the solvent is a poor solvent for at least one of the metal ion donor and the multidentate ligand.

3. The method according to claim 1 or 2, wherein an amount of the solvent is in a range of 30 to 2000% by weight based on a total amount of the metal ion donor and the multidentate ligand.

4. The method according to any one of claims 1 to 3, wherein the method is carried out at a temperature lower than a normal boiling point of the solvent.

5. The method according to any one of claims 1 to 4, wherein the method is carried out while supplying at least one gas selected from the group consisting of dry air, argon, nitrogen, and oxygen into a reaction vessel.

6. The method according to any one of claims 1 to 5, wherein
the centrifugal force is generated by stirring the formulation by rotating a rotary blade within a reaction vessel, and
the shear force is generated by contact between the formulation and an inner wall of the reaction vessel due to the stirring, or by contact between particles constituting the formulation due to the stirring.

7. The method according to any one of claims 1 to 6, wherein the centrifugal force and the shear force are applied to the formulation by a thin film swirl mixing method.

8. The method according to any one of claims 1 to 7, wherein the Metal-Organic Framework is a Porous Coordination Polymer.
